# EUROPÄISCHE PATENTSCHRIFT

(11) **EP 0 628 818 B1**
(45) Veröffentlichungstag und Bekanntmachung des Hinweises auf die Patenterteilung: **18.08.1999**
(21) Anmeldenummer: 94108184.6
(22) Anmeldetag: 27.05.1994
(51) Int. Cl.: G01N 33/53, G01N 33/543, G01N 33/58, G01N 33/68

(54) **Verfahren zum Nachweis eines programmierten oder induzierten Zelltods eukaryontischer Zellen**
Method for the detection of a programmed or induced cell death of eucaryotic cells
Méthode pour la detection de la mort induite ou programmée des cellules eucaryotes

(30) Priorität: 03.06.1993 DE 4318402
(43) Veröffentlichungstag der Anmeldung: 14.12.1994
(73) Patentinhaber: Roche Diagnostics GmbH, 68298 Mannheim (DE)
(72) Erfinder: Trauth, Bernhard, Dr., D-68167 Mannheim (DE)

(56) Entgegenhaltungen:
- CLINICAL IMMUNOLOGY AND IMMUNOPATHOLOGY, Bd.60, Nr.1, Juli 1991, NEW YORK, NY, US Seiten 13 - 26 D.A. BELL AND B. MORRISON 'The Spontaneous Apoptotic Cell Death of Normal Human Lymphocytes in Vitro: The Release of, and Immunoproliferative Response to Nucleosomes in Vitro'
- ADVANCES IN IMMUNOLOGY, Bd.50, 1991, SAN DIEGO, CA, US Seiten 55 - 85 J. JOHN COHEN 'Programmed Cell Death in the Immune System'
- DATABASE WPI Section Ch, Week 8704, Derwent Publications Ltd., London, GB; Class B04, AN 87-024743 & JP-A-61 280 282 (BEPPU T.) 10. Dezember 1986
- JOURNAL OF IMMUNOLOGICAL METHODS., Bd.145, Nr.1-2, 1991, NEW YORK US Seiten 185 - 192 POLLY MATZINGER 'The JAM test: A simple assay for DNA fragmentation and cell death'
- HISTOCHEMISTRY, Bd.101, 1994, BERLIN, DE Seiten 73 - 78 ICHIRO NARUSE, HIROMI KEINO, YOSHIHIKO KAWARADA 'Antibody against single-stranded DNA detects both programmed cell death and drug-induced apoptosis'

## Beschreibung

Die Erfindung betrifft ein Verfahren zum Nachweis eines programmierten oder induzierten Zelltods eukaryontischer Zellen sowie einen für dieses Nachweisverfahren geeigneten Testkit.

Eine eukaryontische Zelle kann auf zwei grundsätzlich unterschiedlichen Wegen absterben. Ein Weg ist die Nekrose, ein Absterben der Zelle als Folge einer Schädigung, z.B. aufgrund einer unzureichenden Blutzufuhr und damit Unterversorgung mit Sauerstoff und Nährstoffen, oder als Folge einer Vergiftung oder physikalischen Schädigung (mechanische Verletzung, Frost oder Hitze). Der andere Weg, der sogenannte programmierte Zelltod, ist von großer Bedeutung für die Entwicklung und Funktionsfähigkeit von Geweben, Organen und Organismen als Ganzes (J. Cohen, Advances in Immunology 50 (1991), 55 - 85). Die auf diese Weise abgestorbenen Zellen weisen im Zytoplasma mit Histonen assoziierte DNA-Fragmente als Mono- und Oligonukleosome auf. Ein solches Erscheinungsbild wird auch bei einem induzierten Zelltod beobachtet, wie er z.B. durch ionisierende Strahlen (Yamada et al., Int. J. Radiat. Biol. 53 (1988), 65) oder bestimmte monoklonale Antikörper wie z.B. Anti-Fas (Yonehara et al., J. Exp. Med. 169 (1989), 1747 - 1756) oder anti-APO-1 (Trauth et al., Science 245 (1989), 301 - 304) ausgelöst wird. Auch zytotoxische T-Zellen und Natural-Killer-Zellen bewirken einen solchen induzierten Zelltod mit dem beschriebenen Erscheinungsbild (Sanderson, Biol. Rev. 56 (1981), 153 - 197, S. Curnow et al., Cancer Immunol. Immunother. 36 (1993), 149 - 155 und Berke, Immunol. Today 12 (1991), 396 - 399). Sie bewirken jedoch auch eine erhöhte Permeabilität der Plasmamembran, wie sie bei einer Nekrose beobachtet wird (Krähenbühl et al., Immunol. Today 12 (1991), 399 - 402). Die beschriebene Art des programmierten oder induzierten Zelltods wird als Apoptose (Wyllie et al., Int. Rev. of Cytol. 68 (1980), 251 - 301) bezeichnet. Sie ist charakterisiert durch bläschenförmige Ausstülpungen der Plasmamembran, Kondensation des Zytoplasmas und Aktivierung einer endogenen Endonuklease. Im Gegensatz zur Nekrose handelt es sich bei der Apoptose also um einen aktiven Prozeß der eukaryontischen Zelle. Er wird daher auch nicht bei einem Abtöten der Zellen durch Erhitzen, Einfrieren und Auftauen oder Lyse mit einem lytisch wirkenden Antikörper bewirkt (R. Duke et al., Proc. Natl. Acad. Sci. 80 (1983), 6361 - 6365). Die bei der Apoptose aktivierte kalzium- und magnesiumabhängige Endonuklease spaltet den DNA-Doppelstrang in den leicht zugänglichen Linkerregionen zwischen den Nukleosomen in Mono- und Oligonukleosome. Die DNA in den Nukleosomen hingegen ist mit den Core-Histonen H2A, H2B, H3 und H4 eng assoziiert und deshalb geschützt vor der Spaltung durch die Endonuklease (Burgoyne et al., Biochem. J. 143 (1974), 67 und Stach et al., J. Neurochem. 33 (1979), 257). Daher ist nach Extraktion der DNA und Auftrennung im Agarosegel die für apoptotische Zellen typische "DNA-Leiter" zu erkennen, ein Muster von DNA-Fragmenten mit einer Länge von ca. 180 Basenpaaren bzw. einem Vielfachen davon (Wyllie, Nature 284 (1980), 555 - 556 und J. Cohen, Advances in Immunology 50 (1991), 55 - 85). Die Plasmamembran der Zellen bleibt in diesem frühen Stadium der Apoptose intakt. Es kommt zur Anreicherung der Mono- und Oligonukleosomen im Zytoplasma der sterbenden Zelle (Duke et al., Lymphokine Research 5 (1986), 289 - 299).

Zum Nachweis von Apoptose sind eine Reihe von Testverfahren entwickelt worden. Hierzu zählt insbesondere der bereits genannte Nachweis einer "DNA-Leiter" im Agarosegel, der als qualitatives Standardverfahren zum Nachweis einer Apoptose und zur Differenzierung gegenüber einer Nekrose dient. Bei anderen Verfahren wird in der Regel die zu untersuchende Zellpopulation mit ³H- oder ¹²⁵J-markiertem Thymidin vormarkiert und nach Induktion der Apoptose und Zellfraktionierung über Zentrifugationsschritte der Anteil der ³H- oder ¹²⁵J-Markierung in der intakten chromosomalen DNA, der fragmentierten DNA sowie im Zellkulturüberstand bestimmt (R. Duke et al., Proc. Natl. Acad. Sci. 80 (1983), 6361 - 6365 und R. Duke et al., Lymphokine Research 5 (1986), 289 - 299). Eine Vereinfachung dieses Verfahrens stellt der sogenannte JAM-Test dar, bei welchem die Trennung von intakter DNA und DNA-Fragmenten über eine Vakuumfiltration durch Glasfaserfilter erfolgt (P. Matzinger, J. Immunol. Meth. 145 (1991), 185 - 192). Auch dieses Verfahren ist jedoch noch sehr zeitaufwendig und wegen der Verwendung einer radioaktiven Markierung nur in Speziallabors durchführbar. Dies gilt auch für ein weiteres von P. Huang et al. beschriebenes Nachweisverfahren von Apoptose, bei welchem die zelluläre DNA aufgereinigt, nach Dephosphorylierung am 5'-Ende mit ³²P markiert, und im Agarosegel elektrophoretisch aufgetrennt wird. Anschließend wird die Radioaktivität in den einzelnen DNA-Banden bestimmt (Annal. Biochem. 207 (1992), 163 - 167). Ohne eine radioaktive Markierung kommen die z.B. von L. Meyaard et al. oder W. Gorczyca et al. beschriebenen Verfahren aus, bei welchen die durch Apoptose erzeugten DNA-Fragmente über eine Nick-Translation mit biotinyliertem dUTP vormarkiert und dann mit einem fluoreszenzmarkierten Avidin nachgewiesen werden. Bei einem weiteren Verfahren wird nach Auswaschen der zytoplasmatischen, fragmentierten DNA die Kern-DNA mit einem interkalierenden Fluoreszenzfarbstoff wie z.B. Acridin-Orange angefärbt und anschließend die Fluoreszenz gemessen (F. Ojeda, J. Immunol. Meth. 152 (1992), 171 - 176). Diese Verfahren sind jedoch ebenfalls für einen routinemäßigen Zytotoxizitätstest zum Nachweis von Apoptose sehr aufwendig und wegen der zum Teil toxischen Fluoreszenzfarbstoffe sowie des apparativen Aufwands ebenfalls Speziallabors vorbehalten.

Aufgabe der Erfindung war es daher, einen einfachen und schnell durchführbaren Zytotoxizitätstest zum Nachweis von Apoptose zur Verfügung zu stellen.

Diese Aufgabe wird gelöst durch ein Verfahren zum Nachweis eines programmierten oder induzierten Zelltods, welches dadurch gekennzeichnet ist, daß man
a) ein durch Behandlung mit einem nicht-ionischen Detergens erhaltenes Lysat der zu untersuchenden Zellpopulation mit einem Anti-Histon-Antikörper und einem Anti-DNA-Antikörper inkubiert, wobei einer der beiden Antikörper vor oder nach dieser Inkubation an eine feste Phase gebunden wird und der andere Antikörper ein markierter Antikörper ist,
b) feste und flüssige Phase trennt und
c) die Markierung in einer der beiden Phasen als Maß für den programmierten oder induzierten Zelltod bestimmt.

Es hat sich überraschenderweise gezeigt, daß mit diesem einfachen Verfahren durch einen programmierten oder induzierten Zelltod abgestorbene Zellen (apoptotische Zellen) ein Meßsignal ergeben, ohne daß eine Vormarkierung der zu untersuchenden Zellpopulation erforderlich ist. Dadurch können auch in vitro nicht proliferierende Zellen untersucht werden. Das erfindungsgemäße Verfahren erlaubt in einfacher und schnell durchführbarer Weise die quantitative Bestimmung des Ausmaßes der Apoptose in der zu untersuchenden Zellpopulation und ist sehr sensitiv, so daß für die Durchführung des Tests nur geringe Zellmengen (bereits 50 Zellen pro Test ergeben ein Signal, vorzugsweise werden 10³ Zellen pro Test verwendet) benötigt werden.

Zur Freisetzung von zytoplasmatischen DNA-Fragmenten aus apoptotischen Zellen wird die zu untersuchende Zellpopulation mit einem nicht-ionischen Detergens behandelt. Derartige Detergenzien sind dem Fachmann bekannt. Vorzugsweise wird NP-40 (Ethylphenylpolyethylenglycol), Tween 20® (Polyoxyethylensorbitanmonolaurat) oder Triton X-100® (Octylphenolpolyethylenglycolether) verwendet. Diese Detergenzien werden in einer Konzentration von 0,1 % bis 1 % eingesetzt. Die Inkubation der apoptotischen Zellen mit dem Detergens erfolgt bei Raumtemperatur oder 4°C für 10 bis 30 Minuten. Das so erhaltene Lysat wird nach Abtrennung der unlöslichen Bestandteile (Zellkerne, z.B. durch Zentrifugation) mit einem Anti-Histon-Antikörper sowie einem Anti-DNA-Antikörper inkubiert. Dabei wird entweder der Anti-Histon-Antikörper oder der Anti-DNA-Antikörper an eine feste Phase gebunden. Diese Bindung erfolgt entweder vor oder nach der Inkubation des Antikörpers mit dem zytoplasmatischen Zellysat in dem Fachmann bekannter Weise, z.B. über eine direkte adsorptive Bindung an eine feste Phase, durch Bindung eines biotinylierten Antikörpers an eine mit Streptavidin beschichtete feste Phase oder die Bindung an einen in entsprechender Weise immobilisierten Antikörper, der gegen den Anti-Histon-Antikörper bzw. den Anti-DNA-Antikörper gerichtet ist. Als Anti-Histon-Antikörper kann jeder Antikörper verwendet werden, welcher mit einem der nukleosomalen Histone H2A, H2B, H3 und/oder H4 reagiert. Als Anti-DNA-Antikörper kann jeder Antikörper verwendet werden, der an DNA bindet. Derartige Antikörper werden z.B. in Andre-Schwartz et al., Clin. Immunol. Immunopathol. 31 (1984), 261 oder Eilat, Immunol. Today 6 (1985), 123 beschrieben. Sowohl der Anti-Histon-Antikörper als auch der Anti-DNA-Antikörper kann als komplettes Immunglobulin oder auch als funktionelles Fragment eines Immunglobulins wie Fab- oder F(ab')₂-Fragment verwendet werden.

Der nicht zur Immobilisierung an die feste Phase verwendete zweite Antikörper wird zur Markierung verwendet. Dazu kann dieser Antikörper entweder selbst markiert sein oder durch Zugabe eines weiteren markierten Antikörpers, welcher gegen den Anti-DNA-Antikörper bzw. den Anti-Histon-Antikörper gerichtet ist, markiert werden. Die Markierung erfolgt dabei nach dem Fachmann bekannten Verfahren, z.B. über Enzyme, Fluoreszenz- oder Chemilumineszenzfarbstoffe. Die nach Trennung von fester und flüssiger Phase mit der festen Phase assoziierte Markierung ist ein direktes Maß für das Ausmaß der Apoptose in der untersuchten Zellpopulation. Als Negativkontrolle wird jeweils eine Parallelkultur der zu untersuchenden Zellpopulation, in der keine Apoptose induziert wurde, verwendet. Die mit dieser Parallelkultur gemessene, mit der festen Phase assoziierte Markierung liefert den Leerwert für die Bestimmung. Als positiv für das Vorliegen einer Apoptose sind solche Meßwerte für die mit der festen Phase assoziierte Markierung zu werten, die reproduzierbar mindestens um einen Faktor 2 über diesem Leerwert liegen. Als Parallelkultur der zu untersuchenden Zellpopulation, in der keine Apoptose induziert wurde, wird dabei eine Kultur von Zellen des gleichen Zelltyps und der gleichen Herkunft verwendet, bei welcher aufgrund der Vorgeschichte der verwendeten Zellen sowie der Kultivierungsbedingungen davon ausgegangen werden kann, daß keine Apoptose induziert wurde. Für den Fall, daß eine in vitro induzierte Apoptose bestimmt werden soll, wird die zu untersuchende Zellpopulation in zwei Parallelansätze aufgeteilt und dann in Gegenwart bzw. Abwesenheit des Apoptoseinduzierenden Agens (z.B. Camptothecin) vorinkubiert.

In einer bevorzugten Ausführungsform des erfindungsgemäßen Verfahrens wird der zur Immobilisierung verwendete Antikörper zur Bindung an die feste Phase biotinyliert und eine mit Streptavidin beschichtete feste Phase verwendet.

Mit Hilfe des erfindungsgemäßen Verfahrens ist es in einfacher Weise möglich, die Zytotoxizität von Zellen oder Verbindungen, welche eine Apoptose induzieren, zu bestimmen.

Ein weiterer Gegenstand der Erfindung ist daher die Verwendung des erfindungsgemäßen Verfahrens zur Bestimmung der zytotoxischen Wirkung oder Aktivität von zytotoxischen Zellen, Natural-Killer-Zellen, ionisierenden Strahlen oder chemischen Substanzen wie z.B. monoklonalen Antikörpern, Hormonen (z.B. Glucocorticoide) oder Toxinen (z.B. Dioxine). Die zytotoxische Wirkung oder Aktivität ergibt sich dabei aus dem über das erfindungsgemäße Verfahren bestimmten Ausmaß der Apoptose in der als Zielzellen verwendeten Zellpopulation.

Ein weiterer Gegenstand der Erfindung ist ein Reagens-Kit zum Nachweis eines programmierten Zelltods, enthaltend einen Anti-Histon-Antikörper sowie einen Anti-DNA-Antikörper. Vorzugsweise liegt dabei einer der beiden Antikörper in markierter Form vor. Alternativ kann der Kit jedoch auch einen weiteren markierten Antikörper enthalten, welcher entweder gegen den Anti-Histon-Antikörper oder gegen den Anti-DNA-Antikörper gerichtet ist.

Ein bevorzugter Gegenstand der Erfindung ist schließlich ein erfindungsgemäßer Reagens-Kit, bei welchem einer der beiden Antikörper zur Bindung an eine feste Phase in biotinylierter Form vorliegt und welcher zusätzlich eine mit Streptavidin beschichtete feste Phase enthält.

Die Erfindung wird durch das folgende Beispiel näher erläutert.

### Beispiel 1

### Nachweis apoptotischer Zellen

### 1. Induktion der Apoptose

Zellen der humanen promyeloiden leukämischen Zellinie HL60 (ATCC CCL 240) in der exponentiellen Wachstumsphase werden auf eine Zellkonzentration von 10⁵ Zellen pro ml mit Kulturmedium eingestellt und davon je 500 µl mit 500 µl einer Lösung des Topoisomerase I Inhibitors Camptothecin (0-4 µg Camptothecin/ml in Kulturmedium, wobei der Wert 0 µg/ml als Negativkontrolle dient) bei 37°C und 5 % CO₂ für 4 h inkubiert.

### 2. Probenaufbereitung

Nach der Inkubation werden die Zellen bei 200 g für 5 min abzentrifugiert, der Überstand abgesaugt und das Zellpellet in 1 ml Kulturmedium resuspendiert. Nach erneuter Zentrifugation unter gleichen Bedingungen wird das Zellpellet in 500 µl Inkubationspuffer (s. u.) resuspendiert, gut durchmischt und etwa 30 min bei 4°C inkubiert. Das so erhaltene Lysat wird 10 min bei 20.000 g zentrifugiert und 400 µl des Überstands (zytoplasmatische Fraktion) vorsichtig abgenommen, ohne das Pellet (Zellkerne mit hochmolekularer, nicht fragmentierter DNA) aufzuwirbeln. Der so gewonnene Überstand wird für den folgenden Immunoassay 1 : 10 mit Inkubationspuffer vorverdünnt.

### Inkubationspuffer:

PBS pH 7,4 mit 0,2 % Tween 20, 1 % Rinderserum-Albumin und 5 mmol/l EDTA, wobei PBS = 137 mmol/l NaCl; 2,7 mmol/l KCl, 8 mmol/l Na₂HPO₄ und 1,5 mmol/l KH₂PO₄).

### 3. Immunoassay

Zur Bindung des Anti-Histon-Antikörpers an eine Mikrotiterplatte werden jeweils 100 µl Beschichtungslösung (Na₂HCO₃/NaH₂CO₃ 50 mmol/l pH 9,6) je Vertiefung einer Mikrotiterplatte einpipettiert und 1 h bei Raumtemperatur inkubiert. Anschließend wird die Beschichtungslösung sorgfältig abgesaugt und zur Nachbeschichtung 200 µl Inkubationspuffer je Vertiefung der Mikrotiterplatte einpipettiert, 30 min bei Raumtemperatur inkubiert und die Flüssigkeit durch Aussaugen sorgfältig entfernt. Anschließend werden die Vertiefungen 3 x mit je 250 - 300 µl Waschlösung (PBS (siehe unter Pkt. 2) + 0,1 % Tween 20®) gespült. Nach Zugabe von 100 µl Probelösung (siehe Pkt. 2) je Vertiefung der Mikrotiterplatte wird 90 min bei Raumtemperatur inkubiert und dann 3 x wie oben beschrieben gewaschen. Zum Nachweis von gebundenen apoptotischen DNA-Fragmenten aus der Probelösung werden dann 100 µl eines Peroxidase-markierten Anti-DNA-Antikörpers (Boehringer Mannheim GmbH, Nr. 1525751) zugegeben, 90 min bei Raumtemperatur inkubiert und nochmals wie oben beschrieben 3 x gewaschen. Anschließend erfolgt die Zugabe von 100 µl ABTS®-Substratlösung für die Peroxidasereaktion (Boehringer Mannheim GmbH, Katalog-Nr. 756407 und 1204530) und Mischen auf einem Plattenschüttler, bis die Farbentwicklung eine photometrische Auswertung bei 405 nm gegen Substratlösung als Leerwert erlaubt (10 - 20 min). Das Verhältnis der gemessenen Extinktion der Probelösung zu der gemessenen Extinktion für die unter Pkt. 1 genannte Kontrolle (lebende Zellen ohne Induktion einer Apoptose) ergibt einen Anreicherungsfaktor, der ein Maß für den Anteil apoptotischer Zellen in der untersuchten Zellpopulation darstellt.

## Patentansprüche

1. Verfahren zum Nachweis eines programmierten oder induzierten Zelltods, dadurch gekennzeichnet, daß man
a) ein durch Behandlung mit einem nicht-ionischen Detergens erhaltenes Lysat der zu untersuchenden Zelle mit einem Anti-Histon-Antikörper und einem Anti-DNA-Antikörper inkubiert, wobei einer der Antikörper vor oder nach dieser Inkubation an eine feste Phase gebunden wird und der andere Antikörper ein markierter Antikörper ist,
b) feste und flüssige Phase trennt und
c) die Markierung in einer der beiden Phasen als Maß für den programmierten oder induzierten Zelltod bestimmt.

2. Verfahren gemäß Anspruch 1, dadurch gekennzeichnet, daß man den zu immobilisierenden Antikörper zur Bindung an die feste Phase biotinyliert und eine mit Streptavidin beschichtete feste Phase verwendet.

3. Verwendung eines Verfahrens gemäß einem der Ansprüche 1 oder 2 zur Bestimmung der zytotoxischen Wirkung oder Aktivität von zytotoxischen Zellen, Natural-Killer-Zellen, ionisierender Strahlung oder chemischen Verbindungen wie insbesondere von Antikörpern, Dioxinen oder Camptothecin.

4. Reagens-Kit zum Nachweis eines programmierten Zelltods, enthaltend einen Anti-Histon-Antikörper sowie einen Anti-DNA-Antikörper.

5. Reagens-Kit gemäß Anspruch 4, dadurch gekennzeichnet, daß einer der Antikörper zur Bindung an eine feste Phase in biotinylierter Form vorliegt und der Kit zusätzlich eine mit Streptavidin beschichtete feste Phase enthält.

## Claims

1. Method for the detection of a programmed or induced cell death, wherein
a) a lysate of the cell to be examined obtained by treatment with a non-ionic detergent is incubated with an anti-histone antibody and an anti-DNA antibody, wherein one of the antibodies is bound to a solid phase before or after this incubation and the other antibody is a labelled antibody,
b) the solid and liquid phase are separated and
c) the label is determined in one of the two phases as a measure of the programmed or induced cell death.

2. Method as claimed in claim 1, wherein the antibody to be immobilized is biotinylated for binding to the solid phase and a solid phase coated with streptavidin is used.

3. Use of a method as claimed in one of the claims 1 or 2 for the determination of the cytotoxic effect or activity of cytotoxic cells, natural killer cells, ionizing radiation or chemical compounds such as in particular of antibodies, dioxins or camptothecin.

4. Reagent kit for the detection of a programmed cell death which contains an anti-histone antibody as well as an anti-DNA antibody.

5. Reagent kit as claimed in claim 4, wherein one of the antibodies is present in a biotinylated form for binding to a solid phase and the kit additionally contains a solid phase coated with streptavidin.

## Revendications

1. Procédé de détection d'une mort cellulaire programmée ou induite, caractérisé par le fait
a) qu'un lysat de la cellule à analyser, obtenu par traitement avec un détergent non-ionique, est incubé avec un anticorps anti-histon et un anticorps anti-ADN, l'un des anticorps étant lié à une phase solide avant ou après cette incubation et l'autre anticorps étant un anticorps marqué,
b) que les phases solide et liquide sont séparées, et
c) que le marqueur est déterminé dans l'une des deux phases comme mesure de la mort cellulaire programmée ou induite.

2. Procédé selon la revendication 1, caractérisé par le fait que l'on biotinyle l'anticorps à immobiliser pour le lier à la phase solide et que l'on utilise une phase solide revêtue de streptavidine.

3. Utilisation d'un procédé selon la revendication 1 ou 2 pour la détermination de l'activité cytotoxique ou l'activité de cellules cytotoxiques, de cellules tueuses naturelles, de rayonnements ionisants ou de composés chimiques tels qu'en particulier des anticorps, des dioxines ou la camptothécine.

4. Kit de réactifs destiné à la détection d'une mort cellulaire programmée, contenant un anticorps anti-histon et un anticorps anti-ADN.

5. Kit de réactifs selon la revendication 4, caractérisé par le fait que l'un des anticorps est présent sous forme biotinylée pour être lié à une phase solide et que le kit contient en outre une phase solide revêtue de streptavidine.
